# EUROPÄISCHE PATENTSCHRIFT

(11) **EP 2 910 202 B1**
(45) Veröffentlichungstag und Bekanntmachung des Hinweises auf die Patenterteilung: **25.05.2016**
(21) Anmeldenummer: 15152582.1
(22) Anmeldetag: 27.01.2015
(51) Int. Cl.: A61B 17/29

(54) **Instrument zur Durchführung medizinischer Eingriffe**
Instrument for carrying out medical procedures
Instrument destiné à exécuter des interventions médicales

(30) Priorität: 21.02.2014 DE 102014203168
(43) Veröffentlichungstag der Anmeldung: 26.08.2015
(73) Patentinhaber: Karl Storz GmbH & Co. KG, 78532 Tuttlingen (DE)
(72) Erfinder: Kärcher, Daniel, 78315 Radolfzell (DE); Stefan, Jochen, 88629 Wald (DE)
(74) Vertreter: Ruttensperger, Bernhard

(56) Entgegenhaltungen:
- EP-A1- 2 510 888
- WO-A1-2013/002063
- DE-B3- 10 314 828

## Beschreibung

Die vorliegende Erfindung betrifft ein Instrument zur Durchführung medizinischer Eingriffe, umfassend einen längs einer Schaftlängsachse langge-streckten Rohrschaft, einen Instrumentenkopf, wobei der Instrumentenkopf an dem Rohrschaft um eine Instrumentenkopfschwenkachse schwenkbar getragen ist und wenigstens ein Teil des Instrumentenkopfs um eine Instrumentenkopflängsachse drehbar ist, und eine Radgetriebeanordnung zur Bewegung des Instrumentenkopfs, wobei wenigstens ein Teil des Instrumentenkopfs durch die Radgetriebeanordnung zur Drehung um die Instrumentenkopflängsachse antreibbar ist. Derartige auch als Rohrschaftinstrumente bezeichnete Instrumente werden beispielsweise im Rahmen endoskopischer, minimalinvasiver chirurgischer Eingriffe eingesetzt, um Gewebe zu manipulieren bzw. abzutrennen.

Aus der DE 103 14 828 B3 ist ein mit einem Rohrschaft aufgebautes Instrument bekannt, bei welchem ein Instrumentenkopf im distalen Endbereich des Rohrschafts mit diesem schwenkbar verbunden ist. Ein Teil des Instrumentenkopfs ist um eine zur Achse der Schwenkverbindung des Instrumentenkopfs mit dem Rohrschaft im Wesentlichen orthogonale Drehachse drehbar. Um diesen Teil des Instrumentenkopfs zur Drehung anzutreiben, ist eine als Kegelradgetriebeanordnung ausgebildete Radgetriebeanordnung vorgesehen, welche eine in dem Rohrschaft sich erstreckende Antriebswelle mit einem daran drehfest getragenen Antriebskegelrad, ein mit dem drehbaren Teil des Instrumentenkopfs drehfestes Abtriebskegelrad und ein mit diesen kämmendes Übertragungskegelrad umfasst. Mit dieser Kegelradgetriebeanordnung kann der insbesondere die Werkzeuge für einen chirurgischen Eingriff tragende Teil des Instrumentenkopfs in Rotation versetzt werden. Um den Instrumentenkopf bezüglich des Rohrschafts zu verschwenken, ist in dem Rohrschaft ein Schubrohr angeordnet, welches mit dem am Rohrschaft angelenkten Teil des Instrumentenkopfs über eine Scharnierverbindung gekoppelt ist. Durch Verschiebung des Schubrohrs im Innenraum des Rohrschafts wird eine Betätigungskraft auf den Instrumentenkopf ausgeübt, so dass dieser um eine Schwenkachse bezüglich des Rohrschafts verschwenkt.

Es ist die Aufgabe der vorliegenden Erfindung, ein Instrument zur Durchführung medizinischer Eingriffe vorzusehen, welches bei baulich einfacher Ausgestaltung eine präzise Betätigung ermöglicht.

Erfindungsgemäß wird diese Aufgabe gelöst durch ein Instrument zur Durchführung medizinischer Eingriffe, umfassend einen längs einer Schaftlängsachse langgestreckten Rohrschaft, einen Instrumentenkopf, wobei der Instrumentenkopf an dem Rohrschaft um eine Instrumentenkopfschwenkachse schwenkbar getragen ist und wenigstens ein Teil des Instrumentenkopfs um eine Instrumentenkopflängsachse drehbar ist, und eine Radgetriebeanordnung zur Bewegung des Instrumentenkopfs, wobei wenigstens ein Teil des Instrumentenkopfs durch die Radgetriebeanordnung zur Drehung um die Instrumentenkopflängsachse antreibbar ist.

Dabei ist weiter vorgesehen, dass der Instrumentenkopf durch die Radgetriebeanordnung zur Verschwenkung um die Instrumentenkopfschwenkachse antreibbar ist.

Bei dem erfindungsgemäßen Aufbau wird also der Instrumentenkopf sowohl zur Verschwenkung als auch zur Rotation durch die Radgetriebeanordnung angetrieben, so dass zusätzliche Mechanismen, wie z. B. ein Schubgestänge, zur Erzeugung der Schwenkbewegung nicht erforderlich sind.

Um die beiden Bewegungszustände herbeiführen zu können, wird vorgeschlagen, dass die Radgetriebeanordnung einen ersten Radgetriebezug mit einem ersten Antriebsrad, einem ersten Übertragungsrad und einem ersten Abtriebsrad, sowie einen zweiten Radgetriebezug mit einem zweiten Antriebsrad, einem zweiten Übertragungsrad und einem zweiten Abtriebsrad umfasst. Dabei ist vorteilhafterweise dann vorgesehen, dass das erste Übertragungsrad in Übertragungswechselwirkung mit dem ersten Antriebsrad und dem ersten Abtriebsrad steht, während das zweite Übertragungsrad in Übertragungswechselwirkung mit dem zweiten Antriebsrad und dem zweiten Abtriebsrad steht. Bei dem erfindungsgemäßen Aufbau umfasst also die Radgetriebeanordnung zwei grundsätzlich voneinander unabhängig aufgebaute, jedoch zur Erzeugung der verschiedenen Bewegungszustände zusammenwirkende Radgetriebezüge.

Um eine gemeinsame Einwirkung der beiden Radgetriebezüge auf den Instrumentenkopf in einfacher Art und Weise erreichen zu können, wird vorgeschlagen, dass das erste Abtriebsrad und das zweite Abtriebsrad zur Instrumentenkopflängsachse konzentrisch angeordnet sind oder/und mit dem um die Instrumentenkopflängsachse drehbaren Teil des Instrumentenkopfs drehfest sind.

Auch antriebsseitig kann ein einfach und bei geringer Baugröße zu realisierender, die Zusammenwirkung der beiden Radgetriebezüge ermöglichender Aufbau vorsehen, dass das erste Antriebsrad und das zweite Antriebsrad zur Schaftlängsachse konzentrisch angeordnet und um die Schaftlängsachse drehbar sind. Hierbei kann eine sehr kompakte Bauart dadurch erreicht werden, dass das erste Antriebsrad an einer ersten Antriebswelle drehfest ist, dass das zweite Antriebsrad an einer zweiten Antriebswelle drehfest ist und dass die erste Antriebswelle als die zweite Antriebswelle aufnehmende Höhlwelle ausgebildet ist.

Das erste Übertragungsrad und das zweite Übertragungsrad können so angeordnet sein, dass sie um eine gemeinsame Übertragungsraddrehachse drehbar sind, welche vorteilhafterweise der Instrumentenkopfschwenkachse entsprechen kann. Somit ist gewährleistet, dass unabhängig von der Schwenkposition des Instrumentenkopfs die antriebsseitigen Räder und die abtriebsseitigen Räder immer gleichmäßig mit den Übertragungsrädern zusammenwirken können.

Um einerseits in einfacher Weise den Instrumentenkopf schwenkbar mit dem Rohrschaft zu verbinden, andererseits auch eine definierte Positionierung für die Übertragungsräder erreichen zu können, wird vorgeschlagen, dass wenigstens ein den Instrumentenkopf um die Instrumentenkopfschwenkachse schwenkbar mit dem Rohrschaft verbindender Instrumentenkopfschwenkstift vorgesehen ist und dass das erste Übertragungsrad und das zweite Übertragungsrad auf wenigstens einem Instrumentenkopfschwenkstift drehbar getragen sind.

Die beiden vorangehend angesprochenen Bewegungszustände, also Rotation und Verschwenkung des Instrumentenkopfs bzw. zumindest eines Teils desselben durch die zusammenwirkenden, grundsätzlich aber voneinander unabhängig antreibbaren Radgetriebezüge können in besonders einfacher und vorteilhafter Weise erreicht werden, wenn das erste Übertragungsrad und das zweite Übertragungsrad bezüglich der Instrumentenkopflängsachse oder/und der Schaftlängsachse einander gegenüberliegend angeordnet sind. Insbesondere ermöglicht diese Positionierung auch die parallele Einwirkung der beiden Übertragungsräder auf den Instrumentenkopf zur Erzeugung eines auf den Instrumentenkopf einwirkenden Schwenkdrehmoments.

Eine besonders einfache Betätigung der beiden Radgetriebezüge zur Zusammenwirkung derselben zum Erzeugen der beiden Bewegungszustände kann dadurch erreicht werden, dass der erste Radgetriebezug und der zweite Radgetriebezug das gleiche Drehzahlumsetzverhältnis, vorzugsweise 1:1, aufweisen. Dies ermöglicht es, die beiden Antriebsräder gleichsinnig oder gegensinnig mit gleicher Drehzahl anzutreiben, gleichzeitig aber auch abtriebsseitig die gleiche Drehzahl bei beiden Radgetriebezügen zu erreichen.

Eine bei einfachem Aufbau zuverlässige Drehmomentübertragungswechselwirkung der verschiedenen Räder der Radgetriebeanordnung kann dadurch gewährleistet werden, dass die Radgetriebeanordnung eine Kegelradgetriebeanordnung mit einem ersten Kegelradgetriebezug und einem zweiten Kegelradgetriebezug ist, wobei der erste Kegelradgetriebezug ein erstes Antriebskegelrad, ein erstes Übertragungskegelrad und ein erstes Abtriebskegelrad umfasst und der zweite Kegelradgetriebezug ein zweites Antriebskegelrad, ein zweites Übertragungskegelrad und ein zweites Abtriebskegelrad umfasst. Hierbei kann beispielsweise vorgesehen sein, dass das erste Übertragungskegefrad mit dem ersten Antriebskegelrad einerseits und dem ersten Abtriebskegelrad andererseits in Übertragungswechselwirkung, vorzugsweise in Kämmeingriff steht, während das zweite Übertragungskegelrad mit dem zweiten Antriebskegelrad und dem zweiten Abtriebskegelrad in Übertragungswechselwirkung, vorzugsweise in Kämmeingriff steht.

Um das erfindungsgemäß aufgebaute Instrument bei chirurgischen Eingriffen einsetzen zu können, beispielsweise zur Entnahme von Gewebeproben, kann an dem Instrumentenkopf ein Maulteil um eine Maulteilschwenkachse schwenkbar getragen sein. Zur Betätigung des Maulteils kann ein sich durch den Instrumentenkopf und den Rohrschaft erstreckendes, vorzugsweise flexibles Schwenkbetätigungsgestänge vorgesehen sein. Durch die Flexibilität des Schwenkbetätigungsgestänges wird die Schwenkbetätigung unabhängig von der Schwenkpositionierung des Instrumentenkopfs bezüglich des Rohrschafts gewährleistet.

Zur Hindurchführung des Schwenkbetätigungsgestänges insbesondere durch den Rohrschaft wird vorgeschlagen, dass die zweite Antriebswelle als das Schwenkbetätigungsgestänge aufnehmende Hohlwelle ausgebildet ist. Dabei kann ein gegenseitiges Stören mit einem den Instrumentenkopf an den Rohrschaft anbindenden Instrumentenkopfschwenkstift dadurch vermieden werden, dass dieser eine Durchgangsöffnung für das Schwenkbetätigungsgestänge aufweist.

Zur Betätigung des Instrumentenkopfs, also zur Erlangung der beiden Bewegungszustände Rotation und Verschwenkung, wird weiter vorgeschlagen, dass eine Antriebsanordnung zum Antreiben des Instrumentenkopfs zur Schwenkbewegung um die Instrumentenkopfschwenkachse und zur Drehbewegung um die Instrumentenkopflängsachse vorgesehen ist. Diese Antriebsanordnung kann grundsätzlich ein oder mehrere manuell zu betätigende Betätigungsorgane umfassen, kann aber auch motorisch ausgebildet sein mit einem oder mehreren auf die antriebsseitigen Kegelräder bzw. die diese tragenden Wellen einwirkenden Antriebsmotoren.

Die vorliegende Erfindung wird nachfolgend mit Bezug auf die beiliegenden Figuren detailliert beschrieben. Es zeigt:
- Fig. 1: eine perspektivische Ansicht eines Teils eines Instruments zur Durchführung medizinischer Eingriffe mit zwei Kegelradgetriebezügen;
- Fig. 2: das Instrument der Fig. 1 bei bezüglich eines Rohrschafts verschwenktem Instrumentenkopf;
- Fig. 3: in prinzipartiger Darstellung zwei Kegelradgetriebezüge und deren Zusammenwirkung mit dem Instrumentenkopf.

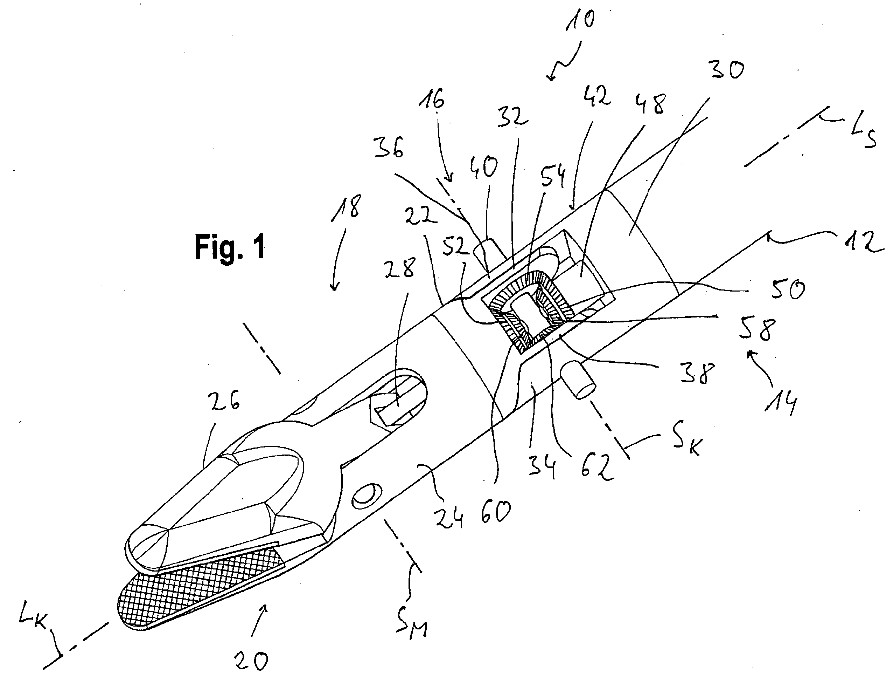

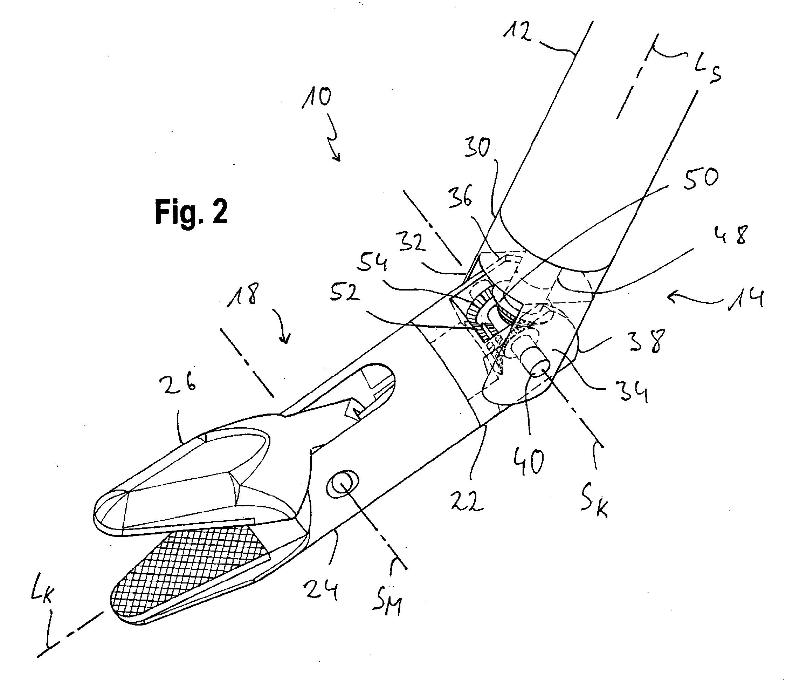

Die Fig. 1 und 2 zeigen ein Instrument, welches zur Durchführung endoskopischer, minimalinvasiver medizinischer Eingriffe eingesetzt werden kann, beispielsweise um Gewebe zu manipulieren bzw. zu entfernen. Das allgemein mit 10 bezeichnete Instrument umfasst einen langgestreckten Rohrschaft 12, der ein- oder mehrteilig ausgebildet sein kann und in seinem distalen Endbereich 14 in einem Gelenkverbindungsbereich 16 mit einem Instrumentenkopf 18 verbunden ist. Der Instrumentenkopf 18 bildet in seiner Gesamtheit den distalen Endbereich 20 des Instruments 10. Der Instrumentenkopf 18 ist mehrteilig aufgebaut mit einem ersten Kopfteil 22, welches in nachfolgend beschriebener Art und Weise im Gelenkverbindungsbereich 16 gelenkig mit dem Rohrschaft 12 verbunden ist. Ein an das erste Kopfteil 22 anschließendes zweites Kopfteil 24 ist zusammen mit dem ersten Kopfteil 22 um eine Instrumentenkopfschwenkachse S_{K} bezüglich des Rohrschafts 12 schwenkbar und ist bezüglich des Rohrschafts 12 bzw. auch bezüglich des ersten Kopfteils 22 um eine Instrumentenkopflängsachse L_{K} drehbar. Am zweiten Kopfteil 24 ist ein Maulteil 26 um eine Maulteilschwenkachse S_{M} schwenkbar getragen. Durch Verschwenken des Maulteils 26 kann dieses zur Entnahme bzw. zur Freigabe beispielsweise einer Gewebeprobe bezüglich des zweiten Kopfteils 24 bewegt werden. Diese Verschwenkbewegung des Maulteils 26 kann durch ein Schwenkbetätigungsgestänge 28 hervorgerufen werden, welches mit dem Maulteil 26 beispielsweise gelenkig verbunden ist und sich durch das Instrument 10, also das Kopfteil 18 sowie den Rohrschaft 12, hindurch erstreckt.

Im Gelenkverbindungsbereich 16 weist der Rohrschaft 12 bzw. ein als distales Endteil 30 desselben ausgebildeter Abschnitt zwei Gelenklaschen 32, 34 auf, die quer zu einer Schaftlängsachse Lₛ, z. B. Längsmittenachse, in Abstand zueinander angeordnet sind. Der Instrumentenkopf 18 bzw. das erste Kopfteil 22 desselben weist in entsprechender Weise zwei Gelenklaschen 36, 38 auf, die quer zu einer Instrumentenkopflängsachse L_{K}, z. B. Längsmittenachse, in Abstand zueinander angeordnet sind, jedoch derart, dass sie innerhalb der beiden Gelenklaschen 32, 34 des Rohrschafts 12 liegen. Ein Instrumentenkopfschwenkstift 40 durchsetzt in den Gelenklaschen 32, 34, 36, 38 vorgesehene und zueinander ausgerichtete Öffnungen. Beispielsweise kann der Instrumentenkopfschwenkstift 40 entweder mit mindestens einer der Gelenklaschen 32, 34 oder mit mindestens einer der Gelenklaschen 36, 38 fest verbunden sein, um ein Herausbewegen des Instrumentenkopfschwenkstifts 40 aus den diesen aufnehmenden Öffnungen zu verhindern. Dasjenige Bauteil, welches mit keiner seiner Gelenklaschen mit dem Instrumentenkopfschwenkstift 40 fest verbunden ist, kann eine Schwenk- bzw. Drehbewegung auf den bezüglich des anderen Bauteils festgehaltenen Instrumentenkopfschwenkstift 40 ausführen. Es sei hier darauf hingewiesen, dass nicht notwendigerweise der Instrumentenkopfschwenkstift 40 in dem dargestellten Ausmaß seitlich über die außen angeordneten Gelenklaschen 32, 34 hervorstehen muss. Um ein Behindern des Eindringens des Instruments 10 in einen Körper zu vermeiden, schließt der Instrumentenkopfschwenkstift 40 vorteilhafterweise im Wesentlichen bündig mit der Außenoberfläche beispielsweise des Rohrschafts 12 ab bzw. steht über diese nicht hervor.

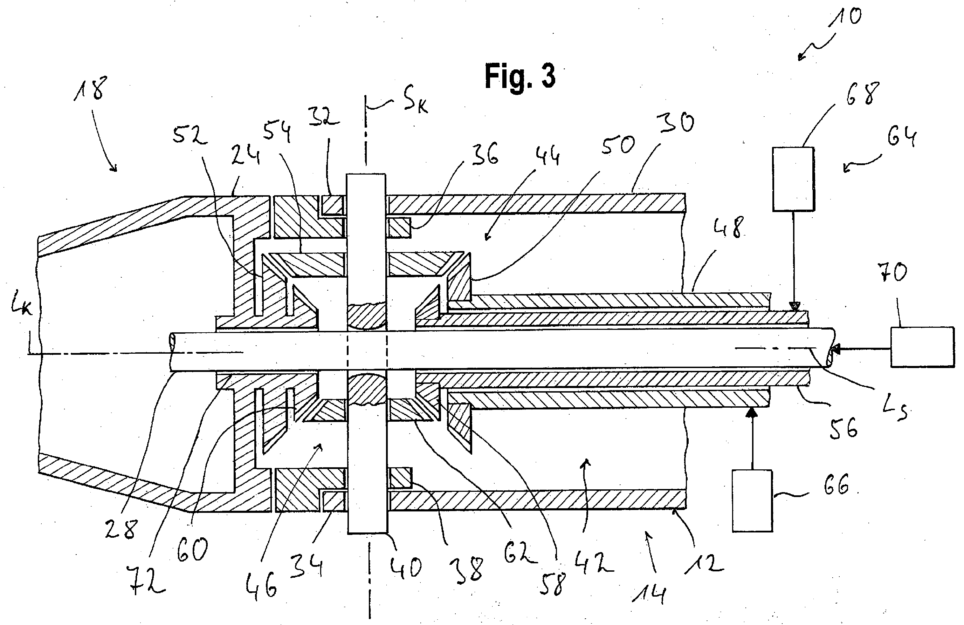

Um die Verschwenkung des Instrumentenkopfs 18 um die Instrumentenkopfschwenkachse Sκ sowie die Rotation des Instrumentenkopfs 18 bzw. des zweiten Kopfteils 24 um die Instrumentenkopflängsachse L_{K} hervorrufen zu können, weist das Instrument 10 eine als Kegelradgetriebeanordnung 42 ausgeführte Radgetriebeanordnung auf. Der Aufbau und die Funktion dieser Kegelradgetriebeanordnung 42 zum Erzeugen der beiden vorangehend angesprochenen Bewegungen wird nachfolgend mit Bezug auf die Fig. 3 detailliert beschrieben.

Die Kegelradgetriebeanordnung 42 umfasst zwei zur Erzeugung der beiden vorangehend angesprochenen Bewegungszustände zusammenwirkende Kegelradgetriebezüge 44, 46. Der erste Kegelradgetriebezug 44 umfasst ein an einer ersten Antriebswelle 48 vorgesehenes erstes Antriebskegelrad 50. Die erste Antriebswelle 48 erstreckt sich vorteilhafterweise koaxial zur Schaftlängsachse Lₛ im Rohrschaft 12 und endet im Gelenkverbindungsbereich 16 vorteilhafterweise derart, dass das daran vorgesehene erste Antriebskegelrad 50 in dem durch die Gelenklaschen 32, 34, 36, 38 freigelassenen Raumbereich angeordnet ist. Es ist in diesem Zusammenhang darauf hinzuweisen, dass die Schaftlängsachse Lₛ im Wesentlichen definiert sein kann durch eine Drehachse der ersten Antriebswelle 48 bzw. des ersten Antriebskegelrads 50 bzw. auch entsprechende Baugruppen des nachfolgend noch erläuterten zweiten Kegelradgetriebezugs 46. Vorteilhafterweise, jedoch nicht zwingend, stimmt eine derartige Drehachse überein mit einer Längsmittenachse des Rohrschafts 12.

Der erste Kegelradgetriebezug 44 umfasst ferner ein erstes Abtriebskegelrad 52. Dieses ist mit dem zweiten Kopfteil 24 drehfest und zur Instrumentenkopfflängsachse L_{κ} zentrisch angeordnet.

Zur Drehmomentübertragung zwischen dem ersten Antriebskegelrad 50 und dem ersten Abtriebskegelrad 52 ist ein erstes Übertragungskegelrad 54 des ersten Kegelradgetriebezugs 44 vorgesehen. Das erste Übertragungskegelrad 54 ist auf dem Instrumentenkopfschwenkstift 40 drehbar getragen und beispielsweise durch Abstützung an der Gelenklasche 36 einerseits und an den beiden Kegelrädern 50, 52 andererseits gegen Verschiebung auf dem Instrumentenkopfschwenkstift 40 gesichert. Selbstverständlich könnten auch unmittelbar am Instrumentenkopfschwenkstift 40 beispielsweise durch Stufen oder Sicherungsringe oder dergleichen Maßnahmen vorgesehen sein, welche eine Verschiebung des ersten Übertragungskegelrads 54 entlang des Instrumentenkopfschwenkstifts 40 verhindern.

Der zweite Kegelradgetriebezug 46 umfasst eine im Rohrschaft 12 sich erstreckende zweite Antriebswelle 56. Diese erstreckt sich innerhalb der als Hohlwelle ausgebildeten ersten Antriebswelle 48 und ist vorteilhafterweise mit dieser im Wesentlichen konzentrisch zur Schaftlängsachse Lₛ angeordnet. In ihrem über die erste Antriebswelle 48 hinaus stehenden Endbereich weist die zweite Antriebswelle 56 ein damit drehfestes zweites Antriebskegelrad 58 auf.

Am Instrumentenkopf 18 bzw. dem zweiten Kopfteil 24 desselben ist in Zuordnung zum zweiten Antriebskegelrad 58 ein zur Instrumentenkopflängsachse L_{K} zentrisch angeordnetes zweites Abtriebskegelrad 60 vorgesehen. Dieses ist somit zusammen mit dem ersten Abtriebskegelrad 52 zur Instrumentenkopflängsachse L_{K} konzentrisch angeordnet und ist überdies mit dem ersten Abtriebskegelrad 52 sowie auch dem zweiten Kopfteil 24 zur gemeinsamen Drehung um die Instrumentenkopflängsachse L_{K} fest verbunden. Diese feste Verbindung kann einerseits durch Ausgestaltung der beiden Abtriebskegelräder 52, 60 und des zweiten Kopfteils 24 als separate Bauteile und das nachfolgende feste Verbinden derselben erreicht werden. Alternativ könnten zumindest zwei dieser Bauteile miteinander integral gefertigt sein. Ferner könnten die beiden Abtriebskegelräder 52, 60 durch verschiedene Kegelabschnitte eines gemeinsamen Abtriebkegelrads bereitgestellt sein.

Der zweite Kegelradgetriebezug 46 umfasst ferner ein zweites Übertragungskegelrad 62. Dieses ist auf dem Instrumentenkopfschwenkstift 40 drehbar getragen. Das zweite Übertragungskegelrad 62 überträgt ein Drehmoment zwischen dem zweiten Antriebskegelrad 58 und dem zweiten Abtriebskegelrad 60. In Zuordnung zu dem zweiten Übertragungskegelrad 62 können am Instrumentenkopfschwenkstift 40 geeignete Maßnahmen vorgesehen sein, um eine Verschiebung des zweiten Übertragungskegelrads 62 auf dem Instrumentenkopfschwenkstift 40 zu verhindern.

Um den Instrumentenkopf über die beiden Kegelradgetriebezüge 44, 46 der Kegelradgetriebeanordnung 48 verschwenken und verdrehen zu können, ist eine allgemein mit 64 bezeichnete Antriebsanordnung vorgesehen. Diese kann beispielsweise an dem nicht dargestellten proximalen Endbereich des Rohrschafts 12 oder einer damit gekoppelten Baugruppe in Zuordnung zu jeder Antriebswelle 48, 56 eine Antriebseinheit 66, 68 aufweisen. Diese Antriebseinheiten 66, 68 können beispielsweise motorisch ausgebildet sein, also beispielsweise jeweils einen Elektroantriebsmotor oder dergleichen umfassen, können gleicherweise aber auch einen einzigen Antriebsmotor umfassen, der über einen Getriebemechanismus auf beide Antriebswellen 48, 56 einwirken kann. Auch ein manuell zu betätigendes Betätigungsorgan in Zuordnung zu jeder der Antriebswellen 48, 56 oder zu beiden Antriebswellen kann zur Betätigung genutzt werden.

Die Betätigung des Maulteils 26 über das Schwenkbetätigungsgestänge 28 kann über eine beispielsweise am proximalen Endbereich des Rohrschafts 12 auf das Schwenkbetätigungsgestänge 28 einwirkende Antriebseinheit 70 erfolgen. Auch diese kann motorisch oder zur manuellen Betätigung durch einen das Instrument 10 nutzenden Artz ausgebildet sein. Um das Schwenkbetätigungsgestänge 28 aus dem Instrumentenkopf 18 in den Bereich des Rohrschafts 12 führen zu können, ist im zweiten Kopfteil 24 vorteilhafterweise konzentrisch zur Instrumentenkopflängsachse L_{κ} eine Durchgriffsöffnung 72 ausgebildet, durch welche hindurch das Schwenkbetätigungsgestänge 28 aus dem Instrumentenkopf 18 heraus geführt ist. Eine entsprechende Durchgriffsöffnung 74 ist auch im Instrumentenkopfschwenkstift 40 vorgesehen. Um eine Verkantung bzw. Zwängung des Schwenkbetätigungsgestänges 28 in der Durchgriffsöffnung 74 bei der Betätigung des Maulteils 26 zu vermeiden, kann diese Durchgriffsöffnung 74 zu ihren beiden Endbereichen hin sich erweiternd ausgebildet sein. Das Schwenkbetätigungsgestänge 28 erstreckt sich dann weiterhin durch die ebenfalls als Hohlwelle ausgebildete zweite Antriebswelle 56, z. B. im Wesentlichen koaxial zur Schaftlängsachse Lₛ, bis in den proximalen Endbereich des Rohrschafts 12. Um die Verschwenkung des Instrumentenkopfs 18 um die Instrumentenkopfschwenkachse S_{A} zu ermöglichen bzw. durch das Schwenkbetätigungsgestänge 28 nicht zu behindern, ist dieses flexibel ausgebildet, so dass es sich insbesondere im Bereich der Hindurchführung durch die Durchgriffsöffnung 74 biegen kann und auch in diesem gebogenen Zustand bei Aktivierung der Antriebseinheit 70 durch die Durchgriffsöffnung 74 hindurch verschoben werden kann. Hierzu kann beispielsweise ein dünner Draht, vorzugsweise Stahldraht, oder auch ein entsprechendes Kunststoffelement als Schwenkbetätigungsgestänge 28 bzw. im Bereich der Gelenkverbindung 16 geführter Abschnitt desselben vorgesehen sein.

Bevor nachfolgend auch mit Bezug auf die Fig. 3 die Funktion der Kegelgetriebeanordnung 42 zur Verschwenkung und Verdrehung des Instrumentenkopfs 18 erläutert wird, sei darauf hingewiesen, dass die vorangehend angesprochenen Kegelräder 50, 52, 54 des ersten Kegelgetrieberadzugs 44 sowie auch die Kegelräder 58, 60, 62 des zweiten Kegelradgetriebezugs 46 zur zuverlässigen Drehmomentübertragung vorteilhafterweise als Zahnräder ausgebildet sind, die in Kämmeingriff miteinander stehen. Dabei ist die Ausgestaltung bzw. Dimensionierung der miteinander in Kämmeingriff stehenden Verzahnungen des ersten Kegelradgetriebezugs 44 einerseits und des zweiten Kegelradgetriebezugs 46 andererseits grundsätzlich voneinander unabhängig. Die Ausgestaltung der Radgetriebeanordnung mit Zahnrädern, diese insbesondere ausgebildet als Kegelräder, ist auf Grund der damit erreichbaren kompakten Bauweise einerseits und der durch den Kämmeingriff der verschiedenen Verzahnungen der Räder bzw. Kegelräder gewährleisteten präzisen Bewegbarkeit des Kopfteils 24 andererseits besonders vorteilhaft. Es sei jedoch darauf hingewiesen, dass die Radgetriebeanordnung mit anderen zur Drehmomentübertragung nutzbaren Rädern ausgebildet sein kann. Beispielsweise können in einem oder beiden Getriebezügen Reibräder, diese beispielsweise auch ausgebildet als Kegelräder, zum Einsatz gelangen. Auch können die verschiedenen Getriebezüge mit Kronrädern oder sonstigen zur Drehmomentübertragung in Wechselwirkung miteinander bringbaren Rädern ausgeführt sein, wobei die beiden Getriebezüge insbesondere auch hinsichtlich der dafür jeweils vorgesehenen Räder unterschiedlich gestaltet sein können.

Um mit der Kegelradgetriebeanordnung 42 eine Rotation des Instrumentenkopfs 18, d. h. des Kopfteils 24 desselben hervorzurufen, werden die beiden Antriebswellen 48, 56 durch die Antriebsanordnung 64 zur Drehung in dergleichen Drehrichtung, vorteilhafterweise auch mit dergleichen Drehzahl, angetrieben. Aufgrund des Umstandes, dass die beiden Übertragungskegelräder 54, 62 bezüglich der Schaftlängsachse Lₛ bzw. auch der Instrumentenkopflängsachse L_{K} einander gegenüberliegend, also an verschiedenen Seiten dieser Achsen angeordnet sind, drehen sich bei dieser gleichsinnigen Rotation der beiden Antriebswellen 48, 56 und damit auch der damit jeweils verbundenen Antriebskegelräder 50, 58 die beiden Übertragungskegelräder 54, 62 zueinander gegensinnig um die Instrumentenkopfschwenkachse S_{K}, welche gleichermaßen die Drehachse dieser beiden Übertragungskegelräder 54, 62 definiert. Eine zueinander gegensinnige Rotation der beiden Übertragungskegelräder 54, 62 führt dazu, dass durch deren Zusammenwirkung mit den Abtriebskegelrädern 52, 60 an bezüglich der Instrumentenkopflängsachse L_{K} entgegengesetzten Seiten diese beiden Abtriebskegelräder 52, 60 zur Rotation in der gleichen Drehrichtung um die Instrumentenkopflängsachse L_{K} angetrieben werden. Dies bedeutet, dass die beiden durch die Kegelradgetriebezüge 44, 46 übertragenen bzw. in den Instrumentenkopf 18 eingeleiteten Antriebsdrehmomente sich addieren und auf diese Art und Weise das zweite Kopfteil 24 zur Drehung um die Instrumentenkopflängsachse L_{K} bezüglich des ersten Kopfteils 22 angetrieben wird.

Um zu verhindern, dass durch diese parallele Wirkung der beiden Kegelradgetriebezüge 44, 46 Zwängungen entstehen bzw. diese Kegelradgetriebezüge 44, 46 zumindest teilweise gegeneinander arbeiten, ist es von Bedeutung, dass die beiden Kegelradgetriebezüge 44, 46 mit demselben Drehzahlumsetzverhältnis arbeiten, unterstellt, dass die beiden Antriebswellen 48, 56 mit gleicher Drehzahl angetrieben werden sollen. Nur so kann erreicht werden, dass bei mit gleicher Drehzahl rotierenden Antriebskegelrädern 50, 58 auch die beiden Abtriebskegelräder 52, 60, welche grundsätzlich drehfest miteinander sind, zur Rotation mit der gleichen Drehgeschwindigkeit um die Instrumentenkopflängsachse L_{K} angetrieben werden. Ein gleiches Drehzahlumsetzverhältnis der beiden Kegelradgetriebezüge 44, 46 kann beispielsweise dadurch realisiert werden, dass die einander jeweils zugeordneten Antriebsund Abtriebskegelräder 50, 52 bzw. 58, 60 gleiche Umsetzverhältnisse generieren, also beispielsweise auch gleiche Durchmesserverhältnisse bereitstellen. Im dargestellten Beispiel ist beim ersten Kegelradgetriebezug 44 das Durchmesserverhältnis und somit das Umsetzverhältnis zwischen Antriebskegelrad 50 und Abtriebskegelrad 52 1:1. Entsprechendes gilt auch für das Antriebskegelrad 58 und das Abtriebskegelrad 60 des zweiten Kegelradgetriebezugs 46. Dies bedeutet, dass im dargestellten Beispiel die Drehbewegung der beiden Antriebswellen 48, 56 1:1 auf den Instrumentenkopf 18 bzw. das zweite Kopfteil 24 desselben übertragen wird. Soll beispielsweise zum Erreichen einer feinfühligeren Rotation des zweiten Kopfteils 24 eine Drehzahluntersetzung stattfinden, könnte dies beispielsweise erreicht werden, indem die beiden Abtriebskegelräder 52, 60 mit größerem Durchmesser ausgebildet werden, als die zugeordneten Antriebskegelräder 50, 58. Dies würde jedoch auch eine entsprechend gestufte Ausgestaltung der Übertragungskegelräder 54 erfordern mit einer ersten Übertragungskegelradstufe zur Zusammenwirkung mit dem jeweiligen Antriebskegelrad und einer zweiten Übertragungskegelradstufe zur Zusammenwirkung mit dem zugeordneten Abtriebskegelrad.

Um mit der Kegelradgetriebeanordnung 42 eine Verschwenkung des Instrumentenkopfs 18 um die Insrumentenkopfschwenkachse S_{κ} zu erzeugen, werden die beiden Antriebswellen 48, 56 durch die Antriebsanordnung 64 vorteilhafterweise mit gleicher Drehzahl zur Drehung in entgegengesetzter Richtung angetrieben. Eine derartige gegensinnige Rotation der beiden Antriebswellen 48, 56 und der daran vorgesehenen Antriebskegelräder 58, 58 hat zur Folge, dass die Übertragungskegelräder 54, 62 zueinander gleichsinnig um die Instrumentenkopfschwenkachse S_{κ} rotieren. Eine gleichsinnige Rotation der beiden Übertragungskegelräder 54, 62 hat zur Folge, dass die beiden miteinander drehfesten Abtriebskegelräder 58, 60 zur Rotation in entgegengesetzter Richtung angetrieben werden. Da dies aufgrund der drehfesten Verbindung dieser beiden Abtriebskegelräder 52, 60 miteinander grundsätzlich nicht möglich ist, entsteht ein durch die Übertragungskegelräder 54, 62 auf den Instrumentenkopf 18 ausgeübtes Drehmoment um die Instrumentenkopfschwenkachse S_{K}, so dass der Instrumentenkopf 18 bei zueinander gegensinniger Rotation der beiden Antriebswellen 48, 56 um die Instrumentenkopfschwenkachse S_{K} schwenkt. Beispielsweise kann bei entsprechendem Antrieb der beiden Antriebswellen 48, 56 ausgehend aus dem in Fig. 1 dargestellten Zustand, in welchem das Instrument 10 nicht abgewinkelt ist und beispielsweise die Schaftlängsachse Lₛ und die Instrumentenkopflängsachse L_{K} zueinander koaxial liegen, eine Verschwenkung in den in Fig. 2 dargestellten Zustand erreicht werden, in welchem der Instrumentenkopf 18 bezüglich des Rohrschafts 12 unter einem von 180° abweichenden Winkel angewinkelt ist und in entsprechendem Maße auch die Instrumentenkopflängsachse L_{K} zur Schaftlängsachse Lₛ angewinkelt ist. In diesem Zustand schneiden sich diese beiden Achsen L_{K} und Lₛ z. B. in der Instrumentenkopfschwenkachse S_{K}.

Aufgrund der vorangehend bereits angesprochenen Flexibilität des Schwenkbetätigungsgestänges 28 für das Maulteil 26 zumindest im Gelenkverbindungsbereich 16 ist auch in diesem bezüglich des Rohrschafts 12 verschwenkten Zustand des Instrumentenkopfs 18 eine Schwenkbetätigung des Maulteils 26 zur Entnahme bzw. auch zur Freigabe von Gewebeproben oder dergleichen möglich.

Mit dem vorangehend beschriebenen Aufbau der Kegelgetriebeanordnung 42 mit ihren beiden sowohl zur Verschwenkung als auch zur Drehung des Kopfteils 18 zusammenwirkenden Kegelradgetriebezügen 44, 46 wird bei kompakter und einfach zu realisierender Ausgestaltung eine präzise Bewegbarkeit des Instrumentenkopfs 18 gewährleistet. Durch die Zusammenwirkung der beiden Kegelradgetriebezüge 44, 46 ist des Weiteren sichergestellt, dass die beiden unterschiedlichen Bewegungen des Instrumentenkopfs 18, d.h. die Rotation desselben einerseits und die Verschwenkung andererseits, einander nicht gegenseitig beeinflussen. Bei Durchführung einer Rotationsbewegung durch entsprechenden Antrieb der beiden Antriebswellen 48, 56 mit jeweils geeigneter Drehzahl bzw. Drehrichtung wird keine Verschwenkung erzeugt. Gleichermaßen wird bei Durchführung einer Verschwenkung durch Antreiben der beiden Antriebswellen 48, 56 mit geeigneter Drehzahl bzw. Drehrichtung keine Rotation erzeugt. Dabei ist die Zusammenwirkung der beiden Kegelradgetriebezüge 44, 46 mit jeweils gleichem Drehzahlumsetzverhältnis besonders vorteilhaft, da in diesem Falle die beiden Antriebswellen 48, 56 beispielsweise über einen gemeinsamen Antrieb oder ein gemeinsames Betätigungsorgan, ggf. unter Zwischenschaltung einer Getriebeanordnung, mit gleicher Drehzahl gleichsinnig oder gegensinnig angetrieben werden können. Die Zusammenwirkung der beiden Kegelradgetriebezüge 44, 46 zur Verdrehung und Verschwenkung des Instrumentenkopfs 18 könnte selbstverständlich auch dann genutzt werden, wenn diese zueinander unterschiedliche Drehzahlumsetzverhältnisse aufweisen. In diesem Falle ist es jedoch vorteilhaft, die beiden Antriebswellen 48, 56 mit entsprechend diesem Unterschied im Drehzahlumsetzverhältnis unterschiedlicher Drehzahl anzutreiben. Dies ist besonders dann in einfacher Weise realisierbar, wenn diesen beiden Antriebswellen 48, 56 voneinander unabhängig aktivierbare Antriebseinheiten, beispielsweise elektromotorische Antriebseinheiten, zugeordnet sind.

Es sei des weiteren darauf hingewiesen, dass der erfindungsgemäße Aufbau selbstverständlich auch bei hinsichtlich ihrer Einsetzbarkeit bzw. hinsichtlich ihres grundsätzlichen Aufbaus anders gestalteten Instrumenten eingesetzt werden kann. So könnte selbstverständlich der Instrumentenkopf 18 anders dimensioniert, beispielsweise länger ausgestaltet sein, als in den Figuren dargestellt. Auch ist der prinzipielle Aufbau bzw. die prinzipielle Funktionsweise der vorliegenden Erfindung unabhängig von der konkreten baulichen Ausgestaltung bzw. Dimensionierung des die beiden Antriebswellen der Kegelradgetriebezüge aufnehmenden Rohrschafts sowie dessen Anbindung an die jeweiligen Antriebseinheiten.

## Patentansprüche

1. Instrument zur Durchführung medizinischer Eingriffe, umfassend:
- einen längs einer Schaftlängsachse (Ls) langgestreckten Rohrschaft (12),
- einen Instrumentenkopf (18), wobei der Instrumentenkopf (18) an dem Rohrschaft (12) um eine Instrumentenkopfschwenkachse (S_{K}) schwenkbar getragen ist und wenigstens ein Teil des Instrumentenkopfs (18) um eine Instrumentenkopflängsachse (L_{K}) drehbar ist,
- eine Radgetriebeanordnung (42) zur Bewegung des Instrumentenkopfs (18), wobei wenigstens ein Teil des Instrumentenkopfs (18) durch die Radgetriebeanordnung (42) zur Drehung um die Instrumentenkopflängsachse (L_{K}) antreibbar ist,
**dadurch gekennzeichnet, dass** der Instrumentenkopf (18) durch die Radgetriebeanordnung (42) zur Verschwenkung um die Instrumentenkopfschwenkachse (S_{K}) antreibbar ist.

2. Instrument nach Anspruch 1,
**dadurch gekennzeichnet, dass** die Radgetriebeanordnung (42) umfasst:
- einen ersten Radgetriebezug (44) mit einem ersten Antriebsrad (50), einem ersten Übertragungsrad (54) und einem ersten Abtriebsrad (52),
- einen zweiten Radgetriebezug (42) mit einem zweiten Antriebsrad (58), einem zweiten Übertragungsrad (62) und einem zweiten Abtriebsrad (60).

3. Instrument nach Anspruch 2,
**dadurch gekennzeichnet, dass** das erste Abtriebsrad (52) und das zweite Abtriebsrad (60) zur Instrumentenkopflängsachse (L_{K}) konzentrisch angeordnet sind oder/und mit dem um die Instrumentenkopflängsachse (L_{K}) drehbaren Teil des Instrumentenkopfs (18) drehfest sind.

4. Instrument nach Anspruch 2 oder 3,
**dadurch gekennzeichnet, dass** das erste Antriebsrad (50) und das zweite Antriebsrad (58) zur Schaftlängsachse (Lₛ) konzentrisch angeordnet und um die Schaftlängsachse (Lₛ) drehbar sind.

5. Instrument nach Anspruch 2, 3 oder 4,
**dadurch gekennzeichnet, dass** das erste Antriebsrad (50) an einer ersten Antriebswelle (48) drehfest ist, dass das zweite Antriebsrad (58) an einer zweiten Antriebswelle (56) drehfest ist und dass die erste Antriebswelle (48) als die zweite Antriebswelle (56) aufnehmende Hohlwelle ausgebildet ist.

6. Instrument nach einem der Ansprüche 2 bis 5,
**dadurch gekennzeichnet, dass** das erste Übertragungsrad (54) und das zweite Übertragungsrad (62) um eine gemeinsame Übertragungsraddrehachse (S_{K}) drehbar sind.

7. Instrument nach Anspruch 6,
**dadurch gekennzeichnet, dass** die Übertragungsraddrehachse (S_{K}) der Instrumentenkopfschwenkachse (S_{K}) entspricht.

8. Instrument nach Anspruch 6 oder 7,
**dadurch gekennzeichnet, dass** wenigstens ein den Instrumentenkopf (18) um die Instrumentenkopfschwenkachse (S_{K}) schwenkbar mit dem Rohrschaft verbindender Instrumentenkopfschwenkstift (40) vorgesehen ist und dass das erste Übertragungsrad (54) und das zweite Übertragungsrad (62) auf wenigstens einem Instrumentenkopfschwenkstift (40) drehbar getragen sind.

9. Instrument nach einem der Ansprüche 6 bis 8,
**dadurch gekennzeichnet, dass** das erste Übertragungsrad (54) und das zweite Übertragungsrad (62) bezüglich der Instrumentenkopflängsachse (L_{K}) oder/und der Schaftlängsachse (Ls) einander gegenüberliegend angeordnet sind.

10. Instrument nach einem der Ansprüche 2 bis 9,
**dadurch gekennzeichnet, dass** der erste Radgetriebezug (44) und der zweite Radgetriebezug (46) das gleiche Drehzahlumsetzverhältnis, vorzugsweise 1:1, aufweisen.

11. Instrument nach einem der Ansprüche 2 bis 10,
**dadurch gekennzeichnet, dass** die Radgetriebeanordnung (42) eine Kegelradgetriebanordnung (42) mit einem ersten Kegelradgetriebezug (44) und einem zweiten Kegelradgetriebezug (42) ist, wobei der erste Kegelradgetriebezug (44) ein erstes Antriebskegelrad, (50), ein erstes Übertragungskegelrad (54) und ein erstes Abtriebskegelrad (52) umfasst und der zweite Kegelradgetriebezug (42) ein zweites Antriebskegelrad (58), ein zweites Übertragungskegelrad (62) und ein zweites Abtriebskegelrad (60) umfasst.

12. Instrument nach einem der Ansprüche 1 bis 11,
**dadurch gekennzeichnet, dass** an dem Instrumentenkopf (18) ein Maulteil (26) um eine Maulteilschwenkachse (S_{M}) schwenkbar getragen ist und dass dem Maulteil (26) ein sich durch den Instrumentenkopf (18) und den Rohrschaft (12) erstreckendes, vorzugsweise flexibles Schwenkbetätigungsgestänge (28) zugeordnet ist.

13. Instrument nach Anspruch 5 und Anspruch 12,
**dadurch gekennzeichnet, dass** die zweite Antriebswelle (56) als das Schwenkbetätigungsgestänge (28) aufnehmende Hohlwelle ausgebildet ist.

14. Instrument nach Anspruch 8 und Anspruch 12 oder 13,
**dadurch gekennzeichnet, dass** ein Instrumentenkopfschwenkstift (40) eine Durchgangsöffnung (74) für das Schwenkbetätigungsgestänge (28) aufweist.

15. Instrument nach einem der Ansprüche 1 bis 14,
**dadurch gekennzeichnet, dass** eine Antriebsanordnung (64) zum Antreiben des Instrumentenkopfs (18) zur Schwenkbewegung um die Instrumentenkopfschwenkachse (S_{K}) und zur Drehbewegung um die Instrumentenkopflängsachse (L_{K}) vorgesehen ist.

## Claims

1. Instrument for carrying out medical interventions, comprising:
- a tubular shaft (12) extending along a longitudinal axis of the shaft (Lₛ),
- an instrument head (18), said instrument head (18) being carried at said tubular shaft (12) to be pivotable about a pivot axis of the instrument head (S_{K}) and at least part of said instrument head (18) being rotatable about a longitudinal axis of said instrument head (L_{K}),
- a wheel gear arrangement (42) for moving the instrument head (18), wherein at least a part of the instrument head (18) can be driven by the wheel gear arrangement (42) for rotating about said longitudinal axis of said instrument head (L_{K}),
**characterized in that** said instrument head (18) can be driven by the wheel gear arrangement (42) for pivoting about said pivot axis of the instrument head (S_{K}).

2. Instrument according to claim 1,
**characterized by** said wheel gear arrangement (42) comprising:
- a first wheel gear train (44) with a first drive wheel (50), a first transmission wheel (54) and a first output wheel (52),
- a second wheel gear train (42) with a second drive wheel (58), a second transmission wheel (62) and a second output wheel (60).

3. Instrument according to claim 2,
**characterized in that** the first output gear (52) and the second output gear (60) are arranged concentrically in relation to the longitudinal axis of said instrument head (L_{K}) or/and are connected to rotate with the part of the instrument head (18) which can be rotated about said longitudinal axis of the instrument head (L_{K}).

4. Instrument according to claim 2 or 3,
**characterized in that** the first drive wheel (50) and the second drive wheel (58) are arranged concentrically in relation to said longitudinal axis of the shaft (Lₛ) and can be rotated about the longitudinal axis of the shaft (Lₛ).

5. Instrument according to claim 2, 3 or 4,
**characterized in that** the first drive wheel (50) is connected to rotate with a first drive shaft (48), that the second drive wheel (58) is connected to rotate with a second drive shaft (56) and that the first drive shaft (48) is adapted as a hollow shaft receiving the second drive shaft (56).

6. Instrument according to one of claims 2 to 5,
**characterized in that** the first transmission wheel (54) and the second transmission wheel (62) can be rotated about a common transmission rotary axis (S_{K}).

7. Instrument according to claim 6,
**characterized in that** said transmission rotary axis (S_{K}) corresponds to said pivot axis of the instrument head (S_{K}).

8. Instrument according to claim 6 or 7,
**characterized in that** at least one instrument head pivot pin (40) is provided, connecting the instrument head (18) to the tubular shaft so that it can be pivoted about said pivot axis of the instrument head (S_{K}) and **in that** the first transmission wheel (54) and the second transmission wheel (62) are carried rotatably on at least one instrument head pivot pin (40).

9. Instrument according to one of claims 6 to 8,
**characterized in that** the first transmission wheel (54) and the second transmission wheel (62) are arranged opposite each other in relation to the longitudinal axis of said instrument head (L_{K}) or/and the longitudinal axis of the shaft (Lₛ).

10. Instrument according to one of claims 2 to 9,
**characterized in that** the first wheel gear train (44) and the second wheel gear train (46) have the same speed transmission ratio, preferably 1:1.

11. Instrument according to one of claims 2 to 10,
**characterized in that** the wheel gear arrangement (42) is a bevel wheel gear arrangement (42) with a first bevel wheel gear train (44) and a second bevel wheel gear train (42), wherein the first bevel wheel gear train (44) comprises a first drive bevel wheel (50), a first transmission bevel wheel (54) and a first output bevel wheel (52) and **in that** the second bevel wheel gear train (42) comprises a second drive bevel wheel (58), a second transmission bevel wheel (62) and a second output bevel wheel (60).

12. Instrument according to one of claims 1 to 11,
**characterized in that** a jaw part (26) is carried at the instrument head (18) and can be pivoted about a jaw part pivot axis (S_{M}) and **in that** a preferably flexible pivot actuation rod assembly (28) extending through the instrument head (18) and the tubular shaft (12) is associated to the jaw part (26).

13. Instrument according to claim 5 and claim 12,
**characterized in that** the second drive shaft (56) is adapted as a hollow shaft receiving said pivot actuation rod assembly (28).

14. Instrument according to claim 8 and claim 12 or 13,
**characterized in that** an instrument head pivot pin (40) comprises a passage opening (74) for said pivot actuation rod assembly (28).

15. Instrument according to claims 1 to 14,
**characterized in that** a drive arrangement (64) is provided for driving the instrument head (18) for a pivot movement about said instrument head pivot axis (S_{K}) and for a rotational movement about said instrument head longitudinal axis (L_{K}).

## Revendications

1. Instrument destiné à effectuer des interventions médicales, comprenant :
- une tige tubulaire (12) s'étendant le long d'un axe longitudinal de la tige (Ls),
- une tête d'instrument (18), ladite tête d'instrument (18) étant supportée à la tige tubulaire (12) de manière pivotable autour d'un axe de la tête d'instrument (S_{K}) et au moins une partie de la tête d'instrument (18) pouvant être tournée autour d'un axe longitudinal de la tête d'instrument (L_{K}),
- un arrangement d'engrenages de roue (42) pour bouger la tête d'instrument (18), au moins une partie de la tête d'instrument (18) pouvant être entraînée en rotation autour de l'axe longitudinal de la tête d'instrument (L_{K}) par l'arrangement d'engrenages de roue (42),
**caractérisé par** la tête d'instrument (18) pouvant être entraînée en pivotement autour de l'axe de la tête d'instrument (S_{K}) par l'arrangement d'engrenages de roue (42).

2. Instrument selon la revendication 1,
**caractérisé par** l'arrangement d'engrenages de roue (42) comprenant :
- un premier train d'engrenage de roue (44) avec une première roue d'entraînement (50), une première roue de transmission (54) et une première roue de sortie (52),
- un deuxième train d'engrenage de roue (42) avec une deuxième roue d'entraînement (58), une deuxième roue de transmission (62) et une deuxième roue de sortie (60).

3. Instrument selon la revendication 2,
**caractérisé par** la première roue de sortie (52) et la deuxième roue de sortie (60) étant arrangées de manière concentrique par rapport à l'axe longitudinal de la tête d'instrument (L_{K}) ou/et étant fixées en rotation à la partie de la tête d'instrument (18) qui peut être tournée autour de l'axe longitudinal de la tête d'instrument (L_{K}).

4. Instrument selon la revendication 2 ou 3,
**caractérisé par** la première roue d'entraînement (50) et la deuxième roue d'entraînement (58) étant concentrique à l'axe longitudinal de la tige (Ls) et pouvant être tournées autour de l'axe longitudinal de la tige (Ls).

5. Instrument selon la revendication 2, 3 ou 4,
**caractérisé par** la première roue d'entraînement (50) étant fixée en rotation à un premier arbre d'entraînement (48), par la deuxième roue d'entraînement (58) étant fixée en rotation à un deuxième arbre d'entraînement (56) et par le premier arbre d'entraînement (48) étant adapté en tant qu'arbre creux recevant le deuxième arbre d'entraînement (56).

6. Instrument selon une des revendications 2 à 5,
**caractérisé par** la première roue de transmission (54) et la deuxième roue de transmission (62) pouvant être tournées autour d'un axe de rotation de roue de transmission commun (S_{K}).

7. Instrument selon la revendication 6,
**caractérisé par** l'axe de rotation de roue de transmission commun (S_{K}) correspondant à l'axe de pivotement de la tête d'instrument (S_{K}).

8. Instrument selon la revendication 6 ou 7,
**caractérisé par** au moins une broche de pivot de tête d'instrument (40) étant prévue reliant la tête d'instrument (18) de manière pivotable autour de l'axe de pivotement de la tête d'instrument (S_{K}) à la tige tubulaire et par la première roue de transmission (54) et la deuxième roue de transmission (62) étant supporté de manière rotative sur au moins une broche de pivot de tête d'instrument (40).

9. Instrument selon une des revendications 6 à 8,
**caractérisé par** la première roue de transmission (54) et la deuxième roue de transmission (62) étant arrangées de manière opposée l'une à l'autre par rapport à l'axe longitudinal de la tête d'instrument (L_{K}) ou/et par rapport à l'axe longitudinal de la tige (Ls).

10. Instrument selon une des revendications 2 à 9,
**caractérisé par** le premier train d'engrenage de roue (44) et le deuxième train d'engrenage de roue (46) ayant le même rapport de transmission de vitesse, de préférence 1:1.

11. Instrument selon une des revendications 2 à 10,
**caractérisé par** l'arrangement d'engrenages de roue (42) étant un arrangement d'engrenages de roue conique (42) avec un premier train d'engrenage de roue conique (44) et un deuxième train d'engrenage de roue conique (42), le premier train d'engrenage de roue conique (44) comprenant une première roue conique d'entraînement (50), une première roue conique de transmission (54) et une première roue conique de sortie (52) et le deuxième train d'engrenage de roue conique (42) comprenant une deuxième roue conique d'entraînement (58), une deuxième roue conique de transmission (62) et une deuxième roue conique de sortie (60).

12. Instrument selon une des revendications 1 à 11,
**caractérisé par** une section de mâchoire (26) étant supportée de manière pivotable autour de l'axe de section de mâchoire (S_{M}) à la tête d'instrument (18) et par une tringlerie d'actuation de pivotement (28), de préférence flexible, qui s'étend à travers la tête d'instrument (18) et la tige tubulaire (12), étant associée à la section de mâchoire (26).

13. Instrument selon la revendication 5 et la revendication 12,
**caractérisé par** le deuxième arbre d'entraînement (56) étant adapté sous forme d'un arbre creux recevant la tringlerie d'actuation de pivotement (28).

14. Instrument selon la revendication 8 et la revendication 12 ou 13,
**caractérisé par** une broche de pivot de tête d'instrument (40) ayant une ouverture de passage (74) pour la tringlerie d'actuation de pivotement (28).

15. Instrument selon une des revendications 1 à 14,
**caractérisé par** un arrangement d'entraînement (64) étant prévu pour entraîner la tête d'instrument (18) à un mouvement de pivotement autour de l'axe de pivotement de la tête d'instrument (S_{K}) et à un mouvement de rotation autour de l'axe longitudinal de la tête d'instrument (L_{K}).
